# EUROPEAN PATENT APPLICATION

(11) **EP 4 268 840 A2**
(43) Date of publication of application: **01.11.2023**
(21) Application number: 21865363.2
(22) Date of filing: 21.12.2021
(51) Int. Cl.: A61K 38/10, A61P 31/12, A61P 31/14, A61P 31/16, A61P 31/22, C07K 14/47

(54) **CIGB-210 AND ANALOGUES FOR THE TREATMENT OF RESPIRATORY INFECTIONS OF VIRAL ORIGIN**

(30) Priority: 23.12.2020 CU 20200110
(71) Applicant: Centro de Ingeniería Genética y Biotecnología, La Habana 11600 (CU)
(72) Inventor: FERNÁNDEZ ORTEGA, Celia, Berta, Playa La Habana 11300 (CU); RAMÍREZ SUÁREZ, Anna, Caridys, Cerro La Habana 12000 (CU); CASILLAS CASANOVA, Dionne, La Habana 10600 (CU); DUARTE CANO, Carlos, Antonio, Playa La Habana 11300 (CU); UBIETA GÓMEZ, Raimundo, Playa La Habana 11600 (CU); GUILLEN NIETO, Gerardo, Enrique, Playa La Habana 11600 (CU); CABRALES RICO, Ania, La Habana 10800 (CU); ÁLVAREZ PÉREZ, Karen, La Habana 17000 (CU); PERERA GONZÁLEZ, Carmen, Laura, San José de las Lajas Mayabeque (CU); FALCON CAMA, Viviana, Playa La Habana 11 600 (CU); PEREA RODRÍGUEZ, Silvio, Ernesto, Playa La Habana 11600 (CU); RODRIGUEZ MOLTO, María, Pilar, Playa La Habana 11600 (CU); GARAY PÉREZ, Hilda, Elisa, Playa La Habana 11600 (CU)
(74) Representative: V.O.
(86) International application number: PCT/CU2021/050015
(87) International publication number: WO 2022/135622

(57) **Abstract**

Peptides that possess an amino acid sequence selected from the group consisting of SEQ ID NO: 2 to SEQ ID NO: 20, as well as pharmaceutical compositions comprising said peptides. Pharmaceutical composition for the treatment or prevention of infections caused by viruses that infect epithelial cells of the mammalian respiratory system comprising a peptide that has an amino acid sequence identified as SEQ ID NO: 1. The invention includes the use of peptides that have an amino acid sequence identified as SEQ ID NO: 1 to SEQ ID NO: 20 for the manufacture of a medicament for the treatment or prevention of infections caused by viruses that infect epithelial cells of the mammalian respiratory system. Also disclosed is a combination of at least one peptide of amino acid sequence identified as SEQ ID NO: 1 to SEQ ID NO: 20 with an antiviral medication.

## Description

### Technical field

The present invention relates to the fields of virology and the pharmaceutical industry. In particular, it discloses a group of synthetic peptides with interrelated sequences that have antiviral activity against the infection of a wide range of viruses that infect epithelial cells of the respiratory system, as well as the synergistic combination of said peptides with other antiviral agents.

### Background of the invention

Acute respiratory diseases are the most frequent ailments in all age groups. An important part of these diseases are caused by viruses, both in humans and in animals, that specifically infect epithelial cells of the respiratory system, either as an initial or subsequent gateway to it. The airway epithelium has morphological and physiological characteristics that distinguish it from the rest of the epithelia. In the first place, the epithelium of most of the respiratory ducts is distinguished by being pseudo stratified, because although it is made up of a single layer of cells, the nuclei are not aligned. On the other hand, unlike the rest of the epithelial cells, it is composed mostly of ciliated cells. The mucus-secreting goblet cells are another cell type present in this epithelium and not in others. The presence of cilia towards the lumen of the respiratory ducts, as well as the action of the goblet cells, maintain the humidity of the ducts and prevent the passage of particles into the lungs. Another distinctive feature of respiratory epithelial cells is the presence of a high number of pattern recognition receptors (PRRs) that are capable of recognizing the so-called PAMPs (Pathogen Associated Molecular Patterns), whose function is to immediately activate the innate immune response in the presence of pathogens. In the respiratory epithelium ten types of Toll-like receptors (TLR1-TLR10) are expressed. Particularly, TLR3, TLR7, TLR8 and TLR9 constitute a family of TLRs specialized in the detection of viruses that infect the respiratory tract. Another property of these cells associated with their defensive functions is their ability to produce molecules with antiviral activity such as interferons, defensins and nitric oxide (NO), among others. In addition, they are capable of secreting adaptive immune response activating cytokines and chemokines (Vareille M et al. 2011, Clin Microbiol Rev, Volume 24. No. 1, p. 210-229).

Among the viruses that enter through the respiratory tract are those of the *Orthomyxoviridae* family, which include influenza viruses; the family *Paramyxoviridae.* comprising parainfluenza, respiratory syncytial virus, and human metapneumoviruses; the *Picornaviridae* family comprising rhinoviruses and coxsackieviruses; the *Adenoviridae* family, which contains the adenoviruses; and the *Coronaviridae* family, which is represented by coronaviruses (Nichols GW et al. 2008 Clin Microbiol Rev, Volume 21, No. 2. p. 274-290; De Clerk and Li. 2016, Clin Microbiol Rev, Volume 29, p. 695-747).

All the viruses mentioned cause diseases in humans and animals. Some of them, such as seasonal influenza, cause large economic losses each year due to their high morbidity, although their fatality is low. However, some influenza variants generated by changes from viruses that infect animals have been the cause of highly lethal outbreaks, such as the Spanish influenza H1N1 of 1918 (40-100 million deaths), the Asian influenza H2N2 of 1957 (1.1 million deaths) or the Hong Kong H3N2 influenza in 1958 (500,000 deaths). In recent years, H5N1 avian influenza, first detected in China in 1996, has caused outbreaks in Asia and North Africa with 50% mortality. Respiratory syncytial virus is estimated to cause 3.2 million hospitalizations and 59,600 deaths among children under 5 years of age, and 118,200 deaths each year (Shi T et al. 2017. Lancet, Volume 390, p. 946-58).

On the other hand, there are viruses that infect their host through other routes and replicate first in other tissues, but during the course of infection they can also infect the respiratory tract causing serious damage. Among this second group is the dengue virus, which, although entering the bloodstream through a mosquito bite, is capable of infecting epithelial lung cells, which causes a worsening of the disease (Cheng NM et al. 2017. PLOS Neglected Tropical Diseases. doi.org/10.1371/journal.pntd.0005520; Lee YR et al. 2007, Virus Res, Volume 126, No. 1, p. 216-25).

The dengue virus infects around 390 million people each year in the world. Of these, 500,000 develop severe illness or hemorrhagic dengue fever, causing 25,000 deaths each year. In fact, many of the severe cases of dengue are related to these respiratory disorders. Some of these lung complications include pulmonary infiltration, pleural effusion, non-cardiogenic pulmonary edema, and respiratory failure (Cheepsattayakorn A and Cheepsattayakorn R. 2014, Journal of Respiratory Medicine Research and Treatment doi: 10.5171/2014.162245; Marchiori E et al. 2009, Orphanet Journal of Rare Diseases, Volume 4, No. 8). Other viruses that present these characteristics are those of the *Herpesviridae* family: Herpes simplex virus (HSV) and cytomegalovirus (CMV). HSV causes nosocomial viral pneumonia, bronchopneumonia, and acute respiratory syndrome (Luyt CE et al. 2011, Presse Med, Volume 40, p. E561-e568).

Antiviral drugs have been developed for some of these viruses that show relative treatment efficacy. For example, among the antivirals that have been approved for use in humans are doxuridine, trifluridine, and brivudine for herpesviruses; oseltamivir, zanamivir, and baloxavir marboxil for influenza, and Palivizumab and Tocozanol for respiratory syncytial virus, Despite this, the continuous development of new more effective antivirals is required, which have new mechanisms of action, lower cost and fewer adverse effects than the existing ones; therefore, the development of new antiviral agents that act through different mechanisms from those already existing is a health priority in the world.

For other viruses, such as dengue and coronaviruses, there are no suitable effective treatments (De Clerk and Li, 2016, Clin Microbiol Rev, Volume 29, p. 695-747). In particular, members of the Coronaviridae family are responsible for a wide spectrum of diseases in animals and humans. SARS-CoV, MERS-CoV and SARS-CoV-2 cause epidemics and serious respiratory infections. The appearance in 2003 of the outbreak of the zoonotic virus SARS-CoV (severe acute respiratory syndrome coronavirus) (Drosten C et al. 2003, New England Journal of Medicine, Volume 348, pp. 1967-1976), and ten years later of the MERS-CoV (Middle East respiratory syndrome coronavirus) (Cui J et al. 2019, Nature Reviews, Volume 17. doi.org/10.1038/s41579-018-0118-9) constituted the first evidence of the great damage caused by this type of virus in humans. Both coronaviruses cause severe respiratory illnesses, infecting nearly 10,000 people before being controlled.

This year, a new coronavirus was reported, SARS-CoV-2, causing the outbreak of the Coronavirus disease 2019 (COVID-19) (Zhou P et al. 2020, Nature, 579, 270-273, doi.org/10.1038/s41586-020-2012-7), which was later declared a COVID-19 pandemic by the World Health Organization. The disease caused by this virus, known as COVID-19, is characterized by an acute respiratory syndrome that can be fatal in about 3% of the patients. This pandemic has spread rapidly around the world, with more than 70 million confirmed cases and 1.5 million deaths, as of November 2020.

There is very little clinical evidence demonstrating the safety and efficacy of any drug against any coronavirus in humans, including SARS-CoV-2 (Kalil AC et al. 2020, JAMA, Volume 323, No. 19, p. 1897-1898). There is also no clinical demonstration of a drug showing antiviral effect in patients with persistent SARS-CoV-2 infection, lasting more than 14 days. An antiviral compound that has shown some therapeutic effect in clinical trials is remdesivir (Wang Y et al. 2020, Lancet, Volume 395, No. 10236, doi.org/10.1016/S0140-6736(20)31022-9), but its scope is limited to a narrow spectrum of patients, and its therapeutic effect is not as powerful as required. The lack of a treatment supported by sufficient scientific evidence has led to the use of different therapeutic regimens and rapid modifications of the protocols. The lack of proven therapies and the need for clinical trials to establish evidence-based treatment guidelines have been underscored (Diaz E et al. 2020, Med Intensiva, doi.org/10.1016/j.medin.2020.06.017). Treatment of SARS-CoV-2 infection remains an unmet medical need. The search for new antiviral therapeutic options for coronavirus infections, in particular SARS-CoV-2, constitutes an urgent need for human health in the world. The threat of new pandemics caused by coronavirus, influenza or other respiratory viruses imposes the need for novel antivirals capable of controlling viral infections.

### Description of the invention

The present invention solves the problem raised above by providing a peptide having an amino acid sequence selected from the group composed by SEQ ID NO: 2 to SEQ ID NO: 20, as well as pharmaceutical compositions comprising at least one of those peptides and a pharmaceutically acceptable excipient. In one embodiment of the invention, said pharmaceutical compositions are formulated for administration by the parenteral or mucosal route. The invention also provides a pharmaceutical composition for the treatment or prevention of infections caused by viruses that infect epithelial cells of the mammalian respiratory system comprising a peptide having an amino acid sequence identified as SEQ ID NO: 1 and a pharmaceutically acceptable excipient. The group of synthetic peptides that are identified as SEQ ID NO: 1 to SEQ ID NO: 20 in the Sequence Listing are related to each other, by their primary structure.

In particular, the invention relates to the inhibition of viral infection originated by viruses that directly infect the epithelial cells of the respiratory system, such as the viruses of the *Coronaviridae. Ortho-* and *Para-myxoviridae. Picornaviridae* and *Adenoviridae* families. More specifically, the invention relates to influenza, parainfluenza, respiratory syncytial virus, coronavirus, enterovirus, and adenovirus viruses, such as SARS-CoV-1. SARS-CoV-2, MERS-CoV, bovine coronavirus (BCoV), influenza A, Parainfluenza type 3, H1N1, H5N1, Adenovirus Ad7, Bocavirus HBoV-1, among others. The invention also relates to the treatment of viral infections caused by the dengue and Zika viruses, herpes simplex viruses and cytomegalovirus, to avoid or treat the serious damages that these viruses can cause at the level of the respiratory tract, mainly the lungs. Also included are viruses that mostly affect animals, such as the classical swine fever virus.

The peptide identified as SEQ ID NO: 1 has been reported previously as an inhibitor of HIV replication, through a modification induced in the vimentin cytoskeleton that prevents viral replication (Fernández-Ortega C et al. 2016, Viruses, Volume 8, No, 6, doi.org/10.3390/v8060098) in cells where the intermediate filaments of the cytoskeleton are formed by vimentin. On the other hand, the state of the art indicated that vimentin was an important target for viral inhibition in cells with cytoskeletal intermediate filaments formed by this protein. In 2015, Zhang et al. reported that vimentin gene silencing affected the viral replication of the TGEV swine coronavirus (Zhang X et al. 2015, Virus Research, http://dx.doi.org/10.1016/j.virusres.2014.12.013).

More recently, vimentin phosphorylation was reported to be modulated in cells infected by SARS-CoV-2 (Bouhaddou M et al. 2020, Cell, volume 182, p. 685-712. https://doi.org/10.1016/j.cell.2020.06.034). However, in the present invention it was found that the peptide identified in the Sequence Listing as SEQ ID NO: 1 exhibits strong antiviral activity against coronaviruses in the main target cells of viral infection, which do not present a vimentin cytoskeleton. Furthermore, it was demonstrated that the antiviral action of this peptide, useful in the treatment of the viral infections mentioned in the present invention, is independent of the vimentin cytoskeleton. More specifically, the inventors show that, surprisingly, this peptide can be used in the treatment of diseases caused by viruses that infect cells in which those skilled in the art do not expect it to have any effect. The present invention also includes peptides that had not been described, which have strong antiviral activity against viruses that infect the epithelial cells of the respiratory system. The first and second examples of the present invention demonstrate the antiviral activity of the peptide identified as SEQ ID NO: 1. and of other peptides related to it, against BCoV and SARS-CoV-2, in cell lines that present intermediate filaments of vimentin as part of their cytoskeleton. Surprisingly, these peptides do not affect the rearrangement of the vimentin cytoskeleton in these cells. Although these cell lines are of epithelial origin, they have developed a vimentin cytoskeleton *in vitro* and. according to the state of the art, its modification due to the action of peptides should be expected, which does not occur. Despite the fact that these cells have vimentin filaments, the peptides of the present invention exhibit inhibitory activity on the viral cytopathic effect, without modification of these cell cytoskeleton filaments. Even more surprising is the demonstration that the peptides identified as SEQ ID NO: 1 to SEQ ID NO: 20 are capable of inhibiting infection by coronavirus and other viruses in primary cultures of epithelial cells of the respiratory system, which do not present vimentin intermediate filaments as part of their cytoskeleton. On the other hand, a previous study reported direct interaction between vimentin and the spike (S) protein of SARS-CoV during viral entry. Furthermore, vimentin is known to play a role in the binding of SARS-CoV to its cellular receptor, and in virus entry into the cell (Yu et al. 2016, Journal of Biomedical Science, volume 23, number 14. doi 10.1186/s12929-016-0234-7). Surprisingly, the peptides of the present invention do not interact with the S protein of SARS-CoV-2, nor with its ACE2 receptor, but with the N protein of said virus.

Another unexpected result of the present invention is that viral inhibition caused by the peptides identified in the Sequence Listing as SEQ ID NO: 1 to 20 was not reached in all the epithelial cells. In the case of the HSV-1 virus, its replication is inhibited efficiently in primary cells of the nasal mucosa by the peptides described. However, it is shown in the examples of this invention that inhibition of viral replication was not achieved in epithelial cells other than those of the respiratory system. All these results, taken together, demonstrate that, very surprisingly, these peptides have antiviral activity against viruses that infect epithelial cells of the respiratory system. This finding refers to, but is not limited to, the inhibition of the replication of SARS-CoV-2, BCoV, Influenza A H1N1, Parainfluenza type 3, Respiratory Syncytial Virus, Adenovirus Ad7, Bocavirus HBoV-1, Dengue virus 2, Herpes simplex HSV-1 and classical swine fever virus.

Therefore, the antiviral action of the peptides of the present invention is exerted through a mechanism that does not involve alterations of the intermediate vimentin filaments nor blocking of the virus binding to its receptor on the target cell membrane. This finding confirms that the peptides of the present invention develop their antiviral activity through novel mechanisms, which have not been reported in the state of the art. Surprisingly, they are capable of inhibiting viral infection in epithelial cells of the respiratory system.

In one embodiment of the invention, the pharmaceutical compositions comprising the peptides identified in the Sequence Listing as SEQ ID NO: 1 to SEQ ID NO: 20 are formulated to be administered by parenteral or mucosal route. In one embodiment of the invention, the infection treated with the peptides SEQ ID NO: 1 to SEQ ID NO: 20 is caused by a virus of the families coronaviridae, ortho- and para-myxoviridae, picornaviridae and adenoviridae. In a particular embodiment, the pharmaceutical composition comprising the peptides SEQ ID NO: 1 to SEQ ID NO: 20 is useful for the treatment or prevention of infections caused by coronavirus, influenza virus, parainfluenza, respiratory syncytial virus, adenovirus, dengue virus, herpes simplex virus, cytomegalovirus and classical swine fever virus. Another object of the invention is the use of a peptide that has an amino acid sequence selected from the group consisting of SEQ ID NO: 1 to SEQ ID NO: 20 for the manufacture of a drug for the treatment or prevention of infections caused by viruses that infect epithelial cells of the mammalian respiratory system. In a particular embodiment, said drug comprises more than one peptide having an amino acid sequence that is selected from the group consisting of SEQ ID NO: 1 to SEQ ID NO: 20.

The present invention, therefore, comprises the use of the peptide of SEQ ID NO: 1 in the treatment of COVID-19, as well as other infections caused by viruses that infect the epithelial cells of the respiratory system. It also provides new peptides structurally related to the peptide of SEQ ID NO: 1 for the treatment of these infections, including those caused by viruses whose initial targets of entry are not cells of the respiratory system, but which can affect cells of this system with more serious clinical consequences. These viruses include herpes viruses, dengue virus, and cytomegalovirus.

In another aspect, the invention provides a method for the treatment or prevention of infections caused by viruses that infect epithelial cells of the mammalian respiratory system characterized in that a therapeutically effective amount of at least one peptide that has an amino acid sequence that is selected from the group consisting of SEQ ID NO: 1 to SEQ ID NO: 20 is administered to an individual in need thereof. In a particular embodiment of the method of the invention, the infection is caused by a virus of the coronaviridae, ortho- and para-myxoviridae, picornaviridae and adenoviridae families. In a preferred embodiment of the invention method, the infection is caused by a virus that is selected from the group consisting of coronavirus, influenza virus, parainfluenza, respiratory syncytial virus, adenovirus, dengue virus, herpes simplex virus, cytomegalovirus and classical swine fever virus.

In one embodiment of the invention, as part of the method of treatment or prevention, an antiviral drug is additionally administered to the individual. The peptide with amino acid sequence that is selected from the group consisting of SEQ ID NO: 1 to SEQ ID NO: 20 and the antiviral drug can be administered simultaneously or sequentially in the same treatment schedule. In one embodiment of the invention, the antiviral medication is selected from the group consisting of Ribavirin, Ivermectin, Penciclovir, Nitazoxanide, Nafamostat, Remdesivir, Favipiravir, the peptide identified as SEQ ID NO: 23, interferon (IFN) alpha, IFN gamma, or a combination of them.

Another object of the invention is a pharmaceutical combination that is characterized by comprising two or more peptides that have an amino acid sequence selected from the group consisting of SEQ ID NO: 1 to SEQ ID NO: 20. In an embodiment of the invention, in the pharmaceutical combination, the peptides are administered simultaneously or sequentially in the course of the same treatment. Furthermore, the invention envisages a pharmaceutical combination for the treatment or prevention of infections caused by viruses that infect epithelial cells of the mammalian respiratory system characterized in that it comprises a) at least one peptide with an amino acid sequence selected from the group consisting of SEQ ID NO: 1 to SEQ ID NO: 20 and b) an antiviral drug. In a particular embodiment, the antiviral drug in the pharmaceutical combination is selected from the group formed by Ribavirin, Ivermectin, Penciclovir, Nitazoxanide, Nafamostat, Remdesivir, Favipiravir, the peptide identified as SEQ ID NO: 23, IFN alpha, IFN gamma, or a combination of them. In such a pharmaceutical combination, the peptide and the antiviral drug are administered simultaneously or sequentially in the course of the same treatment.

In the present invention it is demonstrated that the combination of the peptides of SEQ ID NO: 1 to SEQ ID NO: 20 with other antiviral compounds enhances their inhibitory activity, which results in a synergistic antiviral activity. The antiviral drug is selected from the group formed by Ribavirin, Ivermectin, Penciclovir, Nitazoxanide, Nafamostat, Remdesivir, Favipiravir, the peptide identified as SEQ ID NO: 23, interferon alpha, interferon gamma, or a combination of them, but the scope of the invention is not limited to these.

### Brief description of the figures

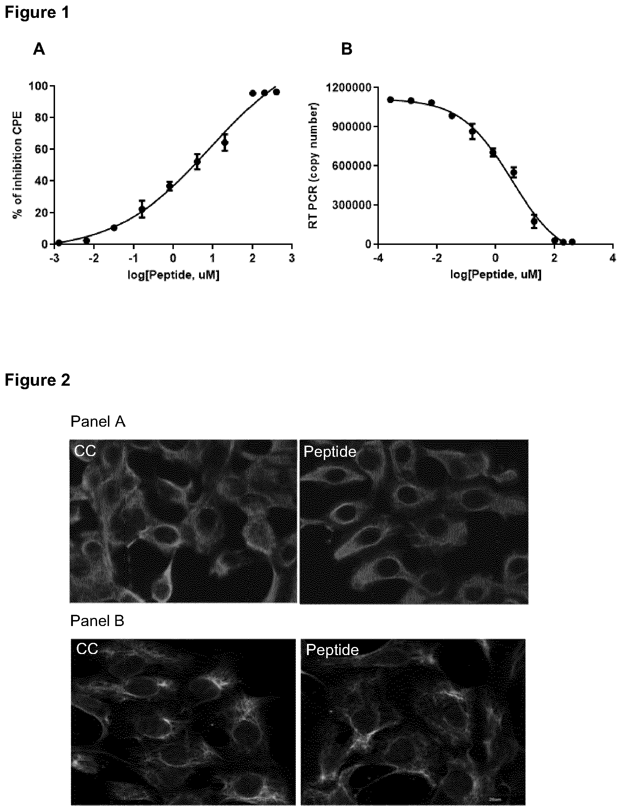

**Figure 1****.** Inhibition of the replication of the Mebus strain of bovine coronavirus by the peptide identified in the Sequence Listing as SEQ ID NO: 1. (**A**): Inhibition of the cytopathic effect (**B**): Reduction in the number of copies of viral ribonucleic acid (RNA) by RT-qPCR.
**Figure 2****.** Immunodetection of vimentin intermediate filaments in cell lines. MDBK cells (**Panel A**) and VeroE6 cells (**Panel B**) were treated with the peptide identified in the Sequence Listing as SEQ ID NO: 1 at 100 µM during 24 h. No differences were observed in the structure of the vimentin filaments between the treated and untreated cells. CC: untreated cells; Target: vimentin cytoskeleton; 40X.

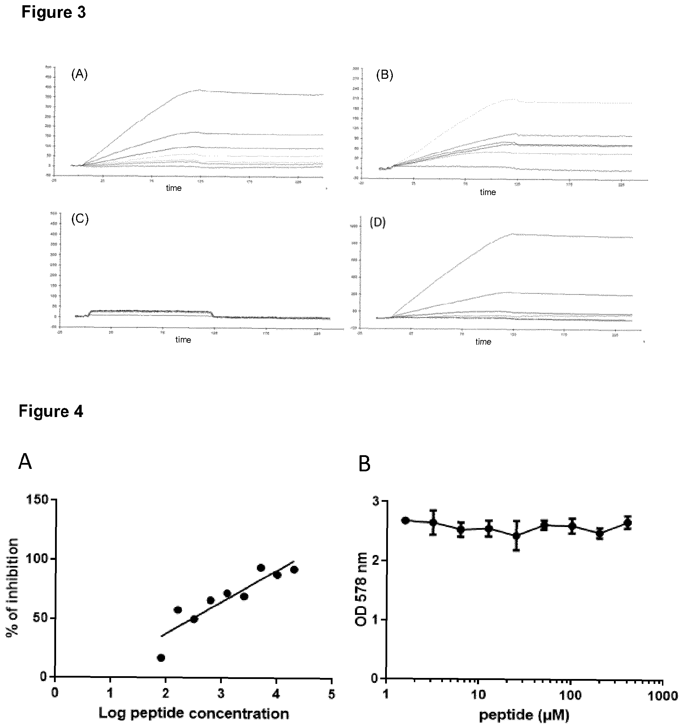

**Figure 3****.** Sensorgrams corresponding to the interaction assays of different molecules with the peptide identified in the Sequence Listing as SEQ ID NO: 1. (**A**) SARS-CoV-2 protein N, (**B**) Dengue 2 virus capsid protein, (**C**) HIV p24 protein, (**D**) Polyclonal anti-SEQ ID NO: 1. All analytes were measured in a concentration range from 10 nM to 200 nM, and at least 5 curves per sample were taken into account to estimate the characteristic parameters of the interaction.
**Figure 4****.** Inhibition of classical swine fever virus by the peptide identified in the Sequence Listing as SEQ ID NO: 1. **A**. Inhibitory effect of viral replication in PK-15 cells. **B**. Cytotoxicity of the peptide in PK15 cells assessed by the 3-(4.5-dimethylthiazol-2-yl)-2,5-diphenyltetrazole (MTT) bromide assay.

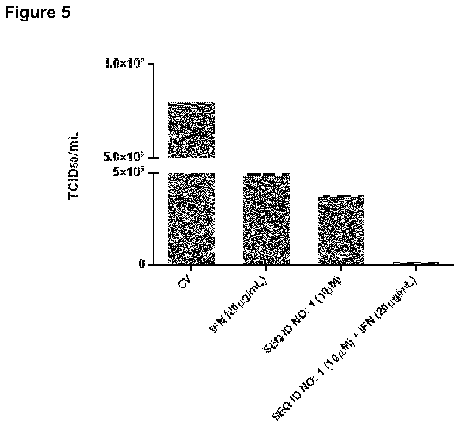

**Figure 5****.** Determination of the antiviral effect of the combined treatment of the peptide identified in the Sequence Listing as SEQ ID NO: 1 and interferon alpha 2b. MDBK cells previously treated during 24 h. with the peptide, interferon or a combination of both were infected with the Mebus strain of bovine coronavirus. The evaluation of the antiviral effect was carried out by determining the infective capacity of the virus present in the supernatant of the cultures 72 hours after infection.

### Examples

### Example 1. Inhibition of bovine coronavirus replication by the peptide identified as SEQ ID NO: 1.

The peptide identified as SEQ ID NO: 1 in the Sequence Listing was obtained by chemical synthesis, using the solid phase synthesis method. To evaluate the antiviral effect of this peptide, bovine kidney cells known as MDBK were seeded in 24-well plates, at 280,000 cells per well, in DMEM culture medium with 10% fetal calf serum (FBS). The plates were incubated in a humid environment at 37 °C and 5% CO₂. After 18 to 24 hours, the peptide identified as SEQ ID NO: 1 was added at different concentrations, and the plate was incubated for 1 hour or 24 hours under the same conditions. Once this treatment time elapsed, the culture medium was removed and the cells were infected with the Mebus strain of bovine coronavirus for 1 hour, in 200 µL of serum-free DMEM medium, at a multiplicity of infection (MOI) of 0.01 in a humid environment at 37 °C and 5% CO₂. Subsequently, the volume of each well was brought up to 1 mL with serum-free DMEM medium in the presence of the peptide. Incubation for 120 hours was carried out, and cell-culture viability was evaluated by the method of metabolic reduction of MTT. To do this, the culture medium was removed. Two-hundred microliters of MTT were added to each well, and the plate was incubated for 2 to 4 hours at 37 °C, 5% CO₂ and 95% humidity. Subsequently, 300 µL of isobutyl alcohol were added and the plate was shaken for 1 hour. As an alternative to the MTT method, cells were stained with crystal violet (0.1%). Finally, the absorbance in each well was determined at 570 nm in a plate reader (Amersham). The antiviral effect was expressed as percent of inhibition of the cytopathic effect (CPE) (Figure 1A) and inhibition of viral RNA (Figure 1B) by RT-qPCR. The results demonstrate that the peptide identified as SEQ ID NO: 1 exerts an antiviral effect on bovine coronavirus infection.

### Example 2. Evaluation of the antiviral effect of the peptides identified as SEQ ID NO: 1 to SEQ ID NO: 22 on the SARS-CoV-2 virus.

The peptides identified as SEQ ID NO: 2 to SEQ ID NO: 22 in the Sequence Listing were obtained by chemical synthesis, using the solid phase synthesis method. To evaluate the antiviral effect of the peptides identified as SEQ ID NO: 1 to SEQ ID NO: 22 against the SARS-CoV-2 coronavirus, the VeroE6 cells (African green monkey kidney cells) were seeded in 96-well plates, at 20,000 cells per well, in MEM culture medium with 10% FBS and incubated in a humid environment at 37 °C and 5% CO₂. After approximately 24 hours elapsed, the evaluated peptides were added in a concentration range from 0.01 to 100 µM and incubated during 1 hour or 24 hours under the same conditions. Subsequently, the culture medium was removed and the cells were infected with the SARS-CoV-2 coronavirus for 1 h, in 100 µL of MEM medium with 2% FBS, at a MOI of 0.01 in a humid environment at 37 °C and 5% CO₂. After that time, the volume of each well was brought up to 200 µL using MEM medium with 2% FBS in the presence of the peptide. The incubation was carried out for 96 hours and the cell viability in the culture was evaluated by the neutral red staining method. For this, the culture medium was removed, 100 µL of the dye were added and incubated for 1 hour at 37 °C, 5% CO₂ and 95% humidity; the absorbance was determined at 580 nm in a plate reader. The antiviral effect was expressed as a percentage of inhibition of the CPE. The results demonstrate that the peptide identified as SEQ ID NO: 1 and some of the related peptides exert an antiviral effect on bovine coronavirus infection as observed in Table 1.

**Table 1. Inhibition of the SARS-CoV-2 virus in cultures treated with the peptides identified as SEQ ID NO: 1 to 22.**

| **Peptide** | **Concentration (µM)** | **Inhibition %** |
|---|---|---|
| Peptide SEQ ID NO: 1 | 50 | 83.3 |
| Peptide SEQ ID NO: 2 | 50 | 95.1 |
| Peptide SEQ ID NO: 3 | 100 | 85.4 |
| Peptide SEQ ID NO: 4 | 100 | 92.4 |
| Peptide SEQ ID NO: 5 | 100 | 85.3 |
| Peptide SEQ ID NO: 6 | 100 | 82.2 |
| Peptide SEQ ID NO: 7 | 100 | 92.4 |
| Peptide SEQ ID NO: 8 | 100 | 90.2 |
| Peptide SEQ ID NO: 9 | 100 | 83.7 |
| Peptide SEQ ID NO: 10 | 100 | 70.8 |
| Peptide SEQ ID NO: 11 | 100 | 73.9 |
| Peptide SEQ ID NO: 12 | 100 | 77.4 |
| Peptide SEQ ID NO: 13 | 100 | 90.5 |
| Peptide SEQ ID NO: 14 | 50 | 83.2 |
| Peptide SEQ ID NO: 15 | 50 | 87.1 |
| Peptide SEQ ID NO: 16 | 100 | 79.6 |
| Peptide SEQ ID NO: 17 | 100 | 72.7 |
| Peptide SEQ ID NO: 18 | 100 | 69.2 |
| Peptide SEQ ID NO: 19 | 50 | 91.8 |
| Peptide SEQ ID NO: 20 | 50 | 69.8 |
| Peptide SEQ ID NO: 21 | 100 | 25.5 |
| Peptide SEQ ID NO: 22 | 100 | 20.2 |

| | | |
|---|---|---|
| Peptide concentration expressed in µM. | | |

### Example 3. Evaluation of the effect of the peptide identified as SEQ ID NO: 1 on the vimentin intermediate filaments in MDBK and VeroE6 cells.

In order to evaluate the effect of the peptide identified as SEQ ID NO: 1 on the vimentin intermediate filaments, MDBK cells and Vero E6 cells were treated with the peptide at 100 µM, during 24 hours at 37 °C and 5% of CO₂. They were then fixed and permeabilized in a solution of acetone:methanol (1:1 V/V) for 10 min at -20 °C and blocked with 1% bovine serum albumin in phosphate buffered saline for 30 min, at 37 °C. Vimentin intermediate filaments were detected using a murine antivimentin IgG1 monoclonal antibody (4.5 µg/mL) followed by an anti-mouse antibody conjugated to fluorescein isothiocyanate (1/200). Vimentin intermediate filaments (FI) were observed distributed throughout the cytoplasm, from the perinuclear region towards one or both poles of the cell. No differences were observed in their supramolecular structure between treated and untreated cells under the evaluation conditions. After treatment, no damage to cell integrity was observed. The vimentin cytoskeleton is not modified by the peptide identified as SEQ ID NO: 1 in MDBK cells (Figure 2A) or in VeroE6 cells (Figure 2B). In similar experiments carried out with the peptides of SEQ ID NO: 2 to 22 no modifications were observed in the structure of the intermediate filaments either.

### Example 4. Anti-SARS-CoV-2 activity exhibited by different peptides in primary cultures of epithelial cells of the respiratory system

Primary epithelial cells obtained from the respiratory tract were used to find out if the peptides under study inhibited the replication of the SARS-CoV-2 virus in cells that do not have vimentin cytoskeleton. These cells are known to have no vimentin intermediate filaments (Robinson-Bennett and Han A 2006. Handbook of Immunohistochemistry and in situ Hybridization of Human Carcinomas, vol, 4. Edited by M.A.Hayat; Elsevier. 537-47). The antiviral activity against the SARS-CoV-2 coronavirus of the peptides identified in the Sequence Listing as SEQ ID NO: 1 to 22 was evaluated in human airway epithelium (HAE), obtained from nasal or bronchial biopsies, or from organ donors, according to Fulcher et al. (Fulcher et al. 2003, Methods in Molecular Medicine, vol. 107, Second Edition, Ed: J. Picot © Humana Press Inc., Totowa, NJ). The primary cells were obtained with the consent of the individuals.

Primary cultures of human airway epithelium were maintained at an air-liquid interface in Transwell plates and infected with SARS-CoV-2 at MOI 1, during 1 hour at 37 °C and 5% CO₂. The cultures were treated prior to viral challenge for 1 or 24 hours at 0.01 to 100 µM concentrations. After the viral challenge, the peptides were replaced in the cultures every 24 hours. After 72 hours, apical washes and SARS-CoV-2 RT-qPCR were performed. Surprisingly, the peptides inhibited the replication of this virus in cells that do not present intermediate vimentin filaments, which indicates an antiviral mechanism independent of this protein (Table 2).

**Table 2. Antiviral activity of various peptides on SARS-CoV-2 replication in primary epithelial cells**

| **Peptide** | **Nasal HAE** | **Bronchial HAE** | | |
|---|---|---|---|---|
| | **Concentration (µM)** | **Inhibition %** | **Concentration (µM)** | **Inhibition %** |
| SEQ ID NO: 1 | 100 | 74 | 100 | 84 |
| SEQ ID NO: 2 | 50 | 80 | 50 | 90 |
| SEQ ID NO: 4 | 100 | 80 | 100 | 85 |
| SEQ ID NO: 8 | 100 | 88 | 100 | 92 |
| SEQ ID NO: 13 | 50 | 79 | 50 | 89 |
| SEQ ID NO: 15 | 50 | 86 | 50 | 90 |
| SEQ ID NO: 19 | 100 | 82 | 100 | 86 |

The remaining peptides identified as SEQ ID NO: 1 to SEQ ID NO: 22 in the Sequence Listing were also evaluated in systems such as the one described, and they inhibited SARS-CoV-2 in primary cultures of human airway epithelium, like in Table 2, with an inhibition range between 60 and 70%, except for the peptides of SEQ ID NO: 21 and 22 that did not reach inhibition percentages higher than 20%.

### Example 5. Interaction of the peptides with SARS-CoV-2 protein N and other viral proteins

From the unexpected results that were obtained, which show that the inhibitory effect of these peptides does not occur through the cytoskeleton, possible routes of action were studied. In particular, the interaction of peptides with SARS-CoV-2 proteins was studied, as well as with some of the proteins involved in the entry of the virus into its human host cells; among them: the nucleocapsid protein of the SARS-CoV-2 virus (N protein), the extracellular domain of the ACE2 receptor and the receptor binding-domain of the viral S protein (RBD). Likewise, the interaction of the peptides with proteins from other viruses was studied, as described below. For this study, a BIACORE type sensor was used, which allows characterizing the interaction between two molecules based on the optical principle called Surface Plasmon Resonance. To perform the necessary measurements, the biotinylated peptide identified as SEQ ID NO: 1 (under medium density conditions) was immobilized on both channels of a streptavidin (SA) functionalized chip (General Electric Healthcare. USA). The fc1 channel was used as the sample channel, while the fc2 channel was used as reference to check the integrity of the immobilized molecule on the surface, after the regeneration processes.

To evaluate the reactivity of the immobilized peptide, a specific polyclonal antibody obtained in rabbits, with a titer of 1/3000 was used against the peptide identified as SEQ ID NO: 1. Application of the polyclonal antibody caused an increase in the signal (RU), an evidence of molecule recognition that can be correlated with its integrity after immobilization on the chip surface (Figure 3D, Table 3).

Analysis of the interaction of the peptide identified as SEQ ID NO: 1 with different proteins revealed that neither the RBD nor the ACE2 peptide interact with the peptide identified as SEQ ID NO: 1. Protein N did show a significant increase in signal (RU) by interacting with the peptide identified as SEQ ID NO: 1 and immobilized in the range of concentrations studied (10-200 nM) (Figure 3A). The K_{D} estimated for this interaction had a value of 3.58 × 10⁻⁹ M, which presupposes high affinity of interaction between both interacting agents (in the nanomolar range).

Figure 3B shows the sensorgram obtained from the study of the interaction of the peptide identified as SEQ ID NO: 1 with the Dengue 2 virus capsid protein, in which a concentration dependent interaction of the protein can be observed. On the other hand, the sensorgram of the interaction of the peptide identified as SEQ ID NO: 1 with the HIV-1 p24 protein (Figure 3C) shows absence of interaction. Other viral proteins with similar characteristics to the SARS-CoV-2 protein N, such as the envelope protein of the Zika virus (ZIKV E), the non-structural protein 1 of the Dengue virus 2 (NS1-D2) and the capsid protein of the Influenza H1N1 virus showed interaction with the peptides identified as SEQ ID NO: 1, 2, 14 and 15. The most important parameters that characterize the interaction of this peptide with the different viral proteins are shown in Tables 3 and 4.

In the case of the HIV-1 p24 protein, there was no interaction with the peptides evaluated (Figure 3C). This result confirms that the inhibition of HIV-1 infection by the already known peptide identified as SEQ ID NO: 1 is caused by a different mechanism of action of this peptide, under certain conditions, which are not fulfilled in epithelial cells of the respiratory system.

**Table 3. Interaction of the peptide identified as SEQ ID NO: 1 with proteins from different viruses and with specific antibody against said peptide.**

| **Sample** | **Rmax (RU)** | **kₐ (1/Mxs)** | **k_{d} (1/s)** | **K_{D} (M)** |
|---|---|---|---|---|
| Protein N (SARS-CoV-2) | 329 | 2.71 x 10⁵ | 9.72 x 10⁻⁴ | 3.58 x 10⁻⁹ |
| pAb anti-SEQ ID NO: 1 | 1.65 x 10⁴ | 2.75 x 10³ | 3.18 x 10⁻⁴ | 1.15 x 10⁻⁷ |
| ZIKV E protein (Zika) | 2.11 x 10⁴ | 9.65 x 10² | 1.54 x 10⁻³ | 1.59 x 10⁻⁶ |
| p24 (HIV) | NI | NI | NI | NI |
| NS1-D2 (Dengue) | 1.86 | 13.3 | 1.02 x 10⁻⁵ | 7.69 x 10⁻⁷ |
| Capsid (Dengue) | 409 | 2.47 x 10⁵ | 4.13 x 10⁻⁴ | 1.67 x 10⁻⁹ |

| | | | | |
|---|---|---|---|---|
| pAb: polyclonal antibody; NI: no interaction; Rmax: maximum response; (RU): resonance units; kₐ (1/Mxs): association rate (1/mol per second); k_{d} (1/s): dissociation rate (1/ second); K_{D} (M): affinity constant (mol) | | | | |

Surprisingly, all these results point to the fact that the antiviral activity of the peptides of the present invention is caused by a direct mechanism of interaction with the virus, independent of the cytoskeleton of the host cell.

**Table 4. Affinity constants (K_{D}) of various related peptides in their interaction with viral proteins.**

| | **Kn (M)** | | | |
|---|---|---|---|---|
| | **Protein N SARS-CoV-2** | **H1N1 Capsid** | **E Protein ZIKV** | **Capsid Dengue 2** |
| Peptide SEQ ID NO: 1 | 3.58 x 10⁻⁹ | 7.69 x 10⁻⁷ | 1.59 x 10⁻⁶ | 1.67 x 10⁻⁹ |
| Peptide SEQ ID NO: 2 | 5.23 x 10⁻⁷ | 2.43 x 10⁻⁸ | 18.2 x 10⁻⁶ | 7.54 x 10⁻⁷ |
| Peptide SEQ ID NO: 14 | 11.4 x 10⁻⁷ | 4.67 x 10⁻⁶ | 3.2 x 10⁻⁶ | 5.91 x 10⁻⁶ |
| Peptide SEQ ID NO: 15 | 7.82 x 10⁻⁶ | 1.89 x 10⁻⁷ | 6.24x 10⁻⁸ | 12.7 x 10⁻⁷ |
| Peptide SEQ ID NO: 21 | NI | NI | NI | NI |

| | | | | |
|---|---|---|---|---|
| NI: No interaction | | | | |

The remaining peptides identified in the Sequence Listing as SEQ ID NO: 1 to SEQ ID NO: 20 were evaluated, with similar results. The peptides identified as SEQ ID NO: 21 and SEQ ID NO: 22 did not interact with the proteins evaluated.

### Example 6. Inhibition of the classical swine fever (CSF) virus by the peptide identified in the Sequence Listing as SEQ ID NO: 1.

In order to evaluate the antiviral effect of the peptide identified in the Sequence Listing as SEQ ID NO: 1, 8500 PK15 cells/well were seeded in 50 µL of DMEM medium in 96-well plates, and 50 µL of DMEM were added, or the peptide diluted in DMEM. Two-fold serial dilutions of the peptide were made starting from 80 µM. Each point was evaluated in triplicate and three virus-free cell controls were included. The plates were incubated for 24 hours at 37 °C and 5% CO₂. Of the CSF virus Margarita strain, 100 TCID50 were added per well (MOI 0.01). The plates were incubated during 72 h, the medium discarded, and the cells fixed for 1 hour at 80 °C. Then they were blocked with 100 µL of 2% milk in PBS for 1 hour at 37 °C. They were washed three times with PBS + 0.05% tween 20 and 100 µL of a 1:3000 dilution of a monoclonal antibody against the E2 viral protein conjugated to peroxidase were added, and incubated for 1 hour at 37 °C. The plates were washed 5 times with PBS + 0.05% tween 20, and the substrate was added (5 mg of *ortho*phenylenediamine plus 5 µL of H₂O₂ in 10 mL of citrate buffer pH 5.0). After 10 min incubation at room temperature, optical density (OD) was determined at 492 nm in an ELISA plate reader.

To obtain the inhibitory concentration 50 (IC₅₀) values, the corresponding doseresponse curves were made (Figure 4), and these values were estimated using the GraphPad Prisma software. In parallel, the cytotoxic effect of the peptide on PK15 cells was studied in a concentration range from 1 to 400 µM by means of the MTT assay. For this, the incubation with the peptide was prolonged during 96 hours, and the development was then carried out by treatment with MTT.

The results obtained demonstrate that the peptide identified as SEQ ID NO: 1 is capable of exerting a concentration-dependent inhibition effect on the replication of the CSF virus (Figure 4A), in a range of doses, in which there is no effect on cell viability (Figure 4B). It was also shown that the antiviral IC₅₀ (300 nM) is much higher than the cytotoxic concentration 50 (CC₅₀ > 400 µM), which gives a very favorable safety index (SI), higher than 1333. The rest of the peptides identified in the Sequence Listing as SEQ ID NO: 2 to SEQ ID NO: 20 were also evaluated in the system described, with similar results.

### Example 7. Demonstration of the antiviral effect of the peptides against different viruses that infect the respiratory tract.

Primary cultures of HAE were obtained from the nasal passages, trachea, bronchi and/or lungs of organ donors, according to Fulcher et al. (Fulcher et al. 2003, Methods in Molecular Medicine, vol. 107, Second Edition. Ed: J. Picot © Humana Press Inc., Totowa, NJ). For the inhibition tests carried out with different respiratory viruses, different groups of the peptides of the present invention were used in order to facilitate the initial demonstrations.

To evaluate the antiviral activity of the peptides identified as SEQ ID NO: 1, 2, 3, 7, 9, 14 and 19 against Influenza H1N1 virus, HAE from tracheo-bronchial tissue were cultured in 12 or 24-well Transwell plates (Costar) and pretreated with the peptides during 2 or 24 hours, at concentrations of 0.01 to 100 µM, before the viral challenge. The cultures were infected with Influenza A H1N1 at 100 PFU (plaque-forming units) for 1 hour at 4 °C, washed with PBS and incubated for 24 hours at 37 °C and 5% CO₂. Then, an apical wash was performed with PBS that was collected for evaluation of viral performance in MDCK cells. To do this, MDCK cells were seeded in 96-well plates and when they reached confluence, they were infected with serial 10-fold dilutions of the apical washes, they were incubated for 1 hour at 37 °C. Next, they were washed with PBS, and a layer of agar in 2x DMEM (1:1) was added and allowed to solidify. Subsequently, the plates were incubated for 48 hours at 37 °C, in 5% CO₂ and stained with crystal violet, for the determination of the number of PFUs. The peptides inhibited viral replication in a percentage range of 67 to 93 (Table 5).

To evaluate the antiviral activity of the peptides identified as SEQ ID NO: 1, 2, 8, 13, 15 and 20 against Parainfluenza virus type 3 (HPIV3), the assay was performed as described for Influenza H1N1, but the infection was carried out with 4000 PFU, during 90 minutes at 37 °C. After washing with PBS, the cultures were incubated for 3 days in the presence of the peptides. Later, an apical wash with PBS was performed and collected, to evaluate viral performance in MDCK cells, as described previously. The results show percentages of viral inhibition between 73 and 90 in presence of the peptides at the concentrations evaluated (Table 5).

The antiviral activity of the peptides identified as SEQ ID NO: 1, 2, 3, 13, 15 and 20 against respiratory syncytial virus (RSV) was evaluated in HAE obtained from bronchi, as described for the Influenza H1N1, but carrying out the infection with 2×10⁶ PFU, for 2 hours at 37 °C. After washing with PBS, the cultures were incubated for 10 days, restoring medium and peptides every 2 days. The virus was assessed by detecting RSV nucleoprotein RNA by RT-qPCR. Virus inhibition values reached more than 75% (75 to 91%) (Table 5).

To evaluate the effect of the peptides identified as SEQ ID NO: 1, 3, 7, 9, 13 and 15 on adenovirus type 7, we proceeded as described for the Influenza H1N1 virus, but infecting the HAE cultures obtained from bronchi with AdV 7 Gomen strain, at an MOI of 1, for 1 hour at 37 °C. The cultures were washed with PBS and incubated for 24 hours at 37 °C and 5% CO₂. Then an apical wash was performed with PBS, which was collected to detect the virus through qPCR. The inhibition values of the virus in presence of the peptides reached more than 68% (68 to 82%) (Table 5).

The effect of the peptides identified as SEQ ID NO: 3, 8, 9, 19 and 20 on human bocavirus (HBoV) was achieved and measured as described for H1N1, using HAE obtained from tracheae, but infecting the cultures with an MOI of 1 at 37 °C for 2 hours. Subsequently, the medium was removed and the cultures were maintained for 72 hours to perform an apical wash and quantify the presence of the virus by qPCR in the NS1 region. Viral inhibition values obtained in the presence of the peptides were between 67 and 87% (Table 5).

To evaluate the inhibition of the peptides identified as SEQ ID NO: 1, 2, 7, 8, 14 and 19 on dengue virus replication, primary cultures of lung cells, obtained from patients with lung carcinoma and maintained in DMEM culture medium with 30% FBS, were carried out. The cultures were treated with the peptides for 2 or 24 hours, at concentrations of 0.01 to 100 µM before viral challenge. The medium was discarded and the cells were infected with Dengue 2 virus, at an MOI of 10 for 2 hours at 37 °C and 5% CO₂. The virus was eliminated, a wash was then performed and the cultures were treated again with peptides in DMEM medium, and incubated for 48 hours at 37 °C and 5% CO₂. Subsequently, the cultures were collected to assess viral performance on VeroE6 cells. For this, the cells were seeded in 24-well plates and when they reached confluence they were infected with serial 10-fold dilutions of the supernatants, incubated for 1 hour at 37 °C and 5% CO₂. Next, a wash was carried out with PBS, and a layer of agar in 2x DMEM (1:1) added. The layer was allowed to solidify and incubated for 48 hours at 37 °C, 5% CO₂. The plates were stained with crystal violet, for the determination of the number of PFUs. The peptides inhibited between 68 and 88% of viral replication (Table 5).

The replication of herpes simplex virus type 1 (HSV-1) was performed in primary explants of human nasal mucosa, according to Glorieux et al. (Glorieux et al. 2011, PLoS ONE, 6(7): e22160, doi: 10.1371/journal.pone.0022160). The explants were cultured in 24-well plates, treated with the peptides identified as SEQ ID NO: 1, 3, 13, 15 and 20, during 2 hours, at concentrations of 0.01 to 100 µM and later infected with 10⁷ TCID₅₀ HSV-1/mL in serum-free medium for 1 hour at 37 °C and 5% CO₂. Afterwards, the cultures were washed and kept at 37 °C and 5% CO₂ for 36 hours, in presence of the peptides in culture medium. The cultures were harvested, and HSV-1 was quantified by qPCR. The percentages of viral inhibition ranged from 70 to 92% in presence of the peptides (Table 5).

All the peptides identified in the Sequence Listing as SEQ ID NO: 1 to 20 were evaluated in subsequent experiments, against each of the mentioned viruses, with similar inhibition ranges to those shown in Table 5.

**Table 5. Antiviral activity of different peptides from the Sequence Listing on the replication of different viruses in primary cultures.**

| **Peptid e SEQ ID NO:** | **Influenza A H1N1** | | **Parainfluenz a Type 3** | | **Sincitial respiratory virus** | | **Adenovirus Ad7** | | **Bocavirus HBoV-1** | | **Dengue 2** | | **Herpes simplex HSV-1** | |
|---|---|---|---|---|---|---|---|---|---|---|---|---|---|---|
| | **Cone. (µM)** | **Inhi b.%** | **Cone. (µM)** | **Inhi b.%.** | **Cone. (µM)** | **Inhi b.%.** | **Cone. (µM)** | **Inhib. %** | **Cone. (µM)** | **Inhi b.%** | **Cone. (µm)** | **Inhib .%** | **Cone. (µM)** | **Inhi b.%** |
| **1** | 50 | 86 | 50 | 79 | 50 | 80 | 50 | 68 | - | - | 100 | 81 | 100 | 92 |
| **2** | 50 | 90 | 50 | 89 | 50 | 83 | - | - | - | - | 50 | 88 | - | - |
| **3** | 50 | 93 | - | - | 50 | 87 | 50 | 82 | 50 | 80 | - | - | 50 | 73 |
| **7** | 50 | 70 | - | - | - | - | 50 | 69 | - | - | 100 | 87 | - | - |
| **8** | - | - | 50 | 73 | - | - | - | - | 50 | 87 | 50 | 72 | - | - |
| **9** | 50 | 89 | - | - | - | - | 50 | 79 | 50 | 75 | - | - | - | - |
| **13** | - | - | 50 | 90 | 50 | 91 | 50 | 76 | - | - | - | - | 50 | 78 |
| **14** | 50 | 79 | - | - | 50 | 82 | - | - | - | - | 50 | 70 | - | - |
| **15** | - | - | 50 | 69 | - | - | 50 | 78 | - | - | - | - | 50 | 81 |
| **19** | 50 | 67 | - | - | 50 | 75 | - | - | 50 | 85 | 50 | 68 | - | - |
| **20** | - | - | 50 | 79 | - | - | - | - | 50 | 67 | - | - | 100 | 70 |

### Example 8. Effect of the peptides on epithelial cells other than those from the respiratory system.

To find out whether the peptides of the present invention inhibited viral infections in primary epithelial cells other than those from the respiratory system, HSV-1 infection was studied in primary cultures of human corneal epithelial cells. The cells were seeded in 48-well plates until they reached 80-90% confluence, they were treated with the peptides identified as SEQ ID NO: 1, 13 and 20, at different concentrations for 2 hours and infected with 10⁷ TCID50 of HSV-1/mL in serum-free medium, for 1 hour at 37 °C and 5% CO₂. The cells were washed, treated again with the peptides, and maintained during 72 hours at 37 °C and 5% CO₂. The presence of HSV-1 in the cultures was determined by qPCR. The percentages of viral inhibition obtained in the presence of the peptides were less than 25%, despite the fact that these peptides were capable of inhibiting this virus in primary respiratory cells as demonstrated in Example 7.

### Example 9. Synergistic effect of the peptide identified as SEQ ID NO: 1 and alpha interferon in the inhibition of bovine coronavirus replication.

The antiviral effect of the peptide identified as SEQ ID NO: 1 in combination with recombinant alfa 2b interferon (Heberon^{™} Alfa R, Cuba) was evaluated by means of the infection system of the Mebus strain of bovine coronavirus, in MDBK cells, using 96-well plates. The peptide treatment was carried out during 24 hours at 10 µM, and the incubation with interferon was carried out for 24 hours at 20 µg/mL. The results are shown in Figure 5. As can be seen, the combination of both molecules reduced viral infection approximately 50 times compared to the peptide alone, and 100 times compared to interferon. The test results demonstrate a potentiation of the antiviral effect of the mentioned peptide by co-treatment with interferon. Similar results were obtained with the peptides identified in the Sequence Listing as SEQ ID NO: 2 to SEQ ID NO: 20.

### Example 10. Effect of the peptides in combination with different compounds that have antiviral activity.

To evaluate the effect of the peptide identified as SEQ ID NO: 1 in combination with compounds that have antiviral activity against SARS-CoV-2 or other viruses, a SARS-CoV-2 viral challenge assay was performed on VeroE6 cells, such as described in Example 2. VeroE6 cells in monolayer were incubated with the peptide identified as SEQ ID NO: 1, at 10 µM during 1 or 24 hours, alone or with each of the antiviral compounds at 37 °C, 5% CO₂, at concentrations from 0.1 to 100 µM. The antiviral effect was expressed as percentage of inhibition of the CPE, and the inhibitory concentration 50 (IC₅₀) of each compound was calculated in the presence or absence of the peptide. In table 6, the compounds assessed in presence of the peptides identified as SEQ ID NO: 1, 2 and 3 show a potentiating effect on the antiviral activity

**Table 6. Inhibitory concentration 50 (IC₅₀) of antiviral compounds combined with the peptides identified in the Sequence Listing as SEQ ID NO: 1, 2 and 3.**

| **Compound** | **IC ₅₀** | | | |
|---|---|---|---|---|
| | **Compound (µM)** | **Compound + peptide SEQ ID NO: 1 (µM)** | **Compound + peptide SEQ ID NO: 2 (µM)** | **Compound + peptide SEQ ID NO: 3 (µM)** |
| Peptide SEQ ID NO: 23 | 3.8 | 0.25 | 0.19 | 0.2 |
| Ribavirin | 40.2 | 7.4 | 6.9 | 7.0 |
| Remdisivir | 2.4 | 0.16 | 0.1 | 0.12 |
| Ivermectin | 2.7 | 0.21 | 0.15 | 0.2 |
| Nafamostat | 20.1 | 1.98 | 1.3 | 1.7 |
| Favipiravir | 65.3 | 12.5 | 9.6 | 10.1 |

The remaining peptides identified in the Sequence Listing as SEQ ID NO: 2 to SEQ ID NO: 20 also enhanced the activity of the compounds mentioned in Table 6.

### Example 11. Pharmaceutical composition that comprises one or more of the peptides identified in the Sequence Listinging as SEQ ID NO: 1 to 20.

In order to have a pharmaceutical composition for the prevention or treatment of infections caused by viruses that affect epithelial cells of the respiratory system, formulations were developed that contained at least one of the peptides identified in the Sequence Listing as SEQ ID NO: 1 to 20 at a concentration of 1.0 to 20.0 mg/mL, sucrose at a concentration between 10.0 to 20.0 mg/ml and acetic acid at 0.1 to 6 µg/ml. The compositions further contained sodium hydroxide and water for injection in qs (concentration sufficient for). The formulations were subjected to an accelerated stability study, and the results showed that they remained as a uniform white lyophilisate, with more than 95% purity, and a microbial limit lower than or equal to 0.01 cfu/mg (colony-forming units/mg), were pyrogen-free, with a humidity percentage lower than or equal to 10%, a peptide content higher than or equal to 60%, and pH between 2 and 6.

### Example 12. Antiviral effect of the peptide identified as SEQ ID NO: 1 in patients infected by SARS-CoV-2.

Human studies were conducted in accordance with the Declaration of Helsinki. A group of patients infected with SARS-CoV-2 were treated with the peptide identified as SEQ ID NO: 1, obtained with a quality fit for clinical use in humans. The peptide was administered subcutaneously. The presence of SARS-CoV-2 viral RNA was determined by RT-qPCR (Roche) in nasopharyngeal swabs of the patients every 48 or 96 hours during 15 days. Four groups were formed, each with 20 patients, all in the initial stage of the disease and with similar characteristics. Group 1 patients received the most frequently used treatment in COVID-19 therapy based on Kaletra (Lopinavir/Ritonavir), chloroquine, and Heberon^{™} Alpha R (IFN α2b). Group 2 received this treatment, plus the peptide identified in the Sequence Listing as SEQ ID NO: 1. The latter was administered subcutaneously, at 2 mg/Kg of weight for 5 days. Group 3 patients received treatment based on Kaletra, chloroquine and a combined formulation of intramuscular IFN α2b (3.0 million international units, MIU) and IFN y (0.5 MIU). Patients in group 4 received the same treatment as Group 3, plus the peptide identified in the Sequence Listing as SEQ ID NO: 1 (2 mg/Kg of weight), subcutaneously for 5 days.

The results showed that patients treated with the peptide became negative for SARS-CoV-2 in a shorter time, compared to those with similar treatments in the absence of peptide. The number of patients who tested negative for SARS-CoV-2 was higher in the groups that received the peptide antiviral treatment (Table 7). On the fourth day after treatment onset, 80 and 85% of the patients treated with the peptide (Groups 2 and 4, respectively) were negative for SARS-CoV-2, higher percentages than the ones reached by the groups that received the treatment without peptide (Groups 1 and 3). On the sixth day after treatment onset, 100% of the patients in Groups 2 and 4 were negative for SARS-CoV-2; while the patients not treated with the peptide reached 100% on day 14 (Table 7).

**Table 7. Effect of the peptide identified as SEQ ID NO: 1 on COVID-19 patients.**

| | **SARS-CoV-2 negative patients/Total** | | | |
|---|---|---|---|---|
| **Days after treatment onset** | **Group 1** | **Group 2** | **Group 3** | **Group 4** |
| **2** | 0/20 | 0/20 | 0/20 | 0/20 |
| **4** | 10/20 | 16/20 | 14/20 | 17/20 |
| **6** | 14/20 | 20/20 | 16/20 | 20/20 |
| **10** | 18/20 | 20/20 | 19/20 | 20/20 |
| **14** | 20/20 | 20/20 | 20/20 | 20/20 |
| **20** | 20/20 | 20/20 | 20/20 | 20/20 |

### Example 13. Effect of the peptide identified in the Sequence Listing as SEQ ID NO: 1 in healthy individuals in contact with COVID-19 patients.

Healthy individuals, contacts of COVID-19 patients, were included in this study. Two groups of 25 healthy individuals exposed to a similar risk of contagion (Group I and Group II) were analyzed. Group II was treated daily, for 6 days, with the peptide identified in the Sequence Listing as SEQ ID NO: 1 as intranasal drops, at a concentration of 14 µg/mL. Nasopharyngeal swab samples were collected for SARS-CoV-2 detection by RT-qPCR (Roche). Sampling was carried out at the beginning of the study and 7 days after it began. The results of the SARS-CoV-2 determinations showed that the individuals treated with the peptide identified in the Sequence Listing as SEQ ID NO: 1 were protected from viral infection, with only one individual infected of the 25 studied (3.9%); in contrast to the untreated group, in which 12 individuals of the 25 studied (48%) were infected with SARS-CoV-2 (Table 8).

**Table 8. Prophylactic effect of the peptide identified in the Sequence Listing as SEQ ID NO: 1 on healthy individuals in contact with COVID-19 patients.**

| **Group** | **SARS-CoV-2 positive cases/Total (%)** | |
|---|---|---|
| | **Day of onset** | **Day 7** |
| I | 0/25 (0%) | 12/25 (48%) |
| II | 0/25 (0%) | 1/25 (3.9%) |

## Claims

1. Peptide **characterized by** having an amino acid sequence selected from the group consisting of SEQ ID NO: 2 to SEQ ID NO: 20.

2. Pharmaceutical composition **characterized by** comprising at least one peptide having an amino acid sequence selected from the group consisting of SEQ ID NO: 2 to SEQ ID NO: 20 and a pharmaceutically acceptable excipient.

3. The pharmaceutical composition of claim 2 that additionally comprises a peptide having an amino acid sequence identified as SEQ ID NO: 1.

4. The pharmaceutical composition of claim 2 that is formulated for administration by the parenteral or mucosal route.

5. Pharmaceutical composition for the treatment or prevention of infections caused by viruses that infect epithelial cells of the mammalian respiratory system comprising a peptide having an amino acid sequence identified as SEQ ID NO: 1 and a pharmaceutically acceptable excipient.

6. The pharmaceutical composition of claim 5 that is formulated for administration by the parenteral or mucosal route.

7. The pharmaceutical composition of claim 5 wherein the infection is caused by a virus of the coronaviridae, ortho- and para-myxoviridae, picornaviridae and adenoviridae families.

8. The pharmaceutical composition of claim 5, wherein the infection is caused by a virus from the group consisting of coronavirus, influenza virus, parainfluenza, respiratory syncytial virus, adenovirus, dengue virus, herpes simplex virus, cytomegalovirus, and classical swine fever virus.

9. Use of a peptide having an amino acid sequence selected from the group consisting of SEQ ID NO: 1 to SEQ ID NO: 20 for the manufacture of a medicament for the treatment or prevention of infections caused by viruses that infect epithelial cells of the mammalian respiratory system.

10. The use of claim 9 wherein the infection is caused by a virus of the coronaviridae, ortho- and para-myxoviridae, picornaviridae and adenoviridae families.

11. The use of claim 9 wherein the infection is caused by a virus selected from the group consisting of coronavirus, influenza virus, parainfluenza virus, respiratory syncytial virus, adenovirus, dengue virus, herpes simplex virus, cytomegalovirus, and classical swine fever virus.

12. The use of claim 9 wherein the medicament comprises more than one peptide having an amino acid sequence that is selected from the group consisting of SEQ ID NO: 1 to SEQ ID NO: 20.

13. Method for the treatment or prevention of infections caused by viruses that infect epithelial cells of the mammalian respiratory system **characterized in that** a therapeutically effective amount of at least one peptide having an amino acid sequence selected from the group consisting of SEQ ID NO: 1 to SEQ ID NO: 20 is administered to an individual in need thereof.

14. The method of claim 13, wherein the infection is caused by a virus of the coronaviridae, ortho- and para-myxoviridae, picornaviridae and adenoviridae families.

15. The method of claim 13 wherein the infection is caused by a virus selected from the group consisting of coronavirus, influenza virus, parainfluenza virus, respiratory syncytial virus, adenovirus, dengue virus, herpes simplex virus, cytomegalovirus, and classical swine fever virus.

16. The method of claim 13 wherein an antiviral drug is additionally administered to the individual.

17. The method of claim 16 wherein the peptide and the antiviral drug are administered simultaneously or sequentially.

18. The method of claim 16 wherein the antiviral drug is selected from the group consisting of Ribavirin, Ivermectin, Penciclovir, Nitazoxanide, Nafamostat, Remdesivir, Favipiravir, the peptide identified as SEQ ID NO: 23, alpha interferon (IFN), gamma IFN, or a combination of them.

19. Pharmaceutical combination **characterized by** comprising two or more peptides possessing an amino acid sequence that is selected from the group consisting of SEQ ID NO: 1 to SEQ ID NO: 20.

20. The pharmaceutical combination of claim 19 wherein the peptides are administered simultaneously or sequentially in the course of the same treatment.

21. Pharmaceutical combination for the treatment or prevention of infections caused by viruses that infect epithelial cells of the mammalian respiratory system **characterized by** comprising: a) at least one peptide having an amino acid sequence selected from the group consisting of SEQ ID NO : 1 to SEQ ID NO: 20 and b) an antiviral drug.

22. The pharmaceutical combination of claim 21 wherein the antiviral drug is selected from the group consisting of Ribavirin, Ivermectin, Penciclovir, Nitazoxanide, Nafamostat, Remdesivir, Favipiravir, the peptide identified as SEQ ID NO: 23, alpha interferon (IFN), gamma IFN, or a combination of them.

23. The pharmaceutical combination of claim 21 wherein the peptide and the antiviral drug are administered simultaneously or sequentially in the course of the same treatment.

24. The pharmaceutical combination of claim 21 wherein the infection is caused by a virus of the coronaviridae, ortho- and para-myxoviridae, picornaviridae and adenoviridae families.

25. The pharmaceutical combination of claim 21 wherein the infection is caused by a virus selected from the group consisting of coronavirus, influenza virus, parainfluenza, respiratory syncytial virus, adenovirus, dengue virus, herpes simplex virus, cytomegalovirus, and classical swine fever virus.
